(19) Europäisches Patentamt
European Patent Office
Office européen des brevets

(11) Numéro de publication : **0 388 308 B1**

# (12) FASCICULE DE BREVET EUROPEEN

(45) Date de publication du fascicule du brevet : **11.05.94 Bulletin 94/19**

(51) Int. Cl.$^5$ : **C07H 15/12,** A61K 31/70, A61K 7/48

(21) Numéro de dépôt : **90400701.0**

(22) Date de dépôt : **15.03.90**

(54) **Esters rétinoiques de D-désosamine, leur procédé de préparation et leur utilisation en médecine humaine ou vétérinaire et en cosmétique.**

(30) Priorité : **16.03.89 FR 8903460**

(43) Date de publication de la demande : **19.09.90 Bulletin 90/38**

(45) Mention de la délivrance du brevet : **11.05.94 Bulletin 94/19**

(84) Etats contractants désignés : **AT BE CH DE DK ES FR GB GR IT LI NL SE**

(56) Documents cités :
**EP-A- 0 253 393**
**WO-A-89/00157**
**FR-A- 2 556 348**
**FR-A- 2 598 420**
**US-A- 3 290 284**

(73) Titulaire : **L'OREAL**
**14, Rue Royale**
**F-75008 Paris (FR)**

(72) Inventeur : **Philippe, Michel**
**3, rue de L'Aubépine**
**F-92160 Antony (FR)**
Inventeur : **Sebag, Henri**
**26, rue Erlanger**
**F-75016 Paris (FR)**

(74) Mandataire : **Stalla-Bourdillon, Bernard et al**
**CABINET NONY & CIE 29, rue Cambacérès**
**F-75008 Paris (FR)**

EP 0 388 308 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouveaux esters rétinoïques de D-désosamine, leur procédé de préparation et leur utilisation en médecine humaine et vétérinaire et en cosmétique.

Ces nouveaux esters rétinoïques de D-désosamine trouvent tout particulièrement une application dans des compositions pharmaceutiques ou cosmétiques destinées notamment à combattre une prolifération bactérienne qu'elle soit d'origine infectieuse ou non.

Ils trouvent également une application dans le traitement topique et systémique des affections dermatologiques liées à un désordre de la kératinisation (différenciation - prolifération) et d'affections dermatologiques, ou autres, à composantes inflammatoires et/ou immunoallergiques et dans les maladies de dégénérescence du tissu conjonctif, et peuvent présenter une activité anti-tumorale. En outre ces dérivés peuvent être utilisés dans le traitement de l'atopie, qu'elle soit cutanée ou respiratoire et du psoriasis rhumatoïde.

Ils peuvent aussi présenter un intérêt contre le vieillissement de la peau.

Ils trouvent également une application dans le domaine ophtalmologique, notamment dans le traitement des cornéopathies.

Il a déjà été proposé dans le brevet français n°84.18617 (2.556.348) de nouveaux rétinoïdes qui sont des esters ou amides de l'acide étrétinique et d'un sucre, dans le traitement des néoplasies, du psoriasis et de l'acné, ceux-ci répondant à la formule suivante:

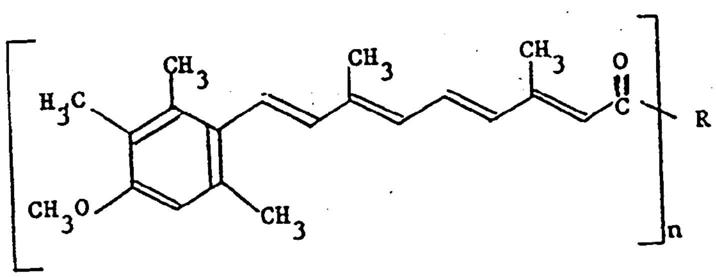

dans laquelle :

R représente le reste d'un sucre relié par une liaison de type ester ou le reste d'un amino sucre relié par une liaison de type amide, ou de dérivés de tels sucres et n est égal à 1 ou 2.

Selon ce brevet, le reste de sucre dérive de préférence du glucose, du maltose, du tréhalose ou du ribose ou encore d'un dérivé de ces sucres.

Après d'importantes études on a constaté de façon tout à fait surprenante et inattendue qu'en utilisant, comme sucre, de la D-désosamine, constituant de divers macrolides dont les érythromycines, pour la formation d'esters non seulement de l'acide étrétinique mais également des acides rétinoïques (all trans) et (13 cis), il était possible de remédier aux inconvénients de ces acides à savoir leur toxicité et notamment leur caractère tératogène.

Les études comparatives réalisées ont par ailleurs permis de mettre en évidence que ces nouvelles propriétés étaient dues essentiellement à la nature du sucre utilisé pour l'estérification à savoir la D-désosamine. En effet les esters correspondant de glucose par exemple ne permettent pas la même détoxification notamment en ce qui concerne la chaîne all-trans.

Par ailleurs, il s'est avéré que ces nouveaux esters rétinoïques de D-désosamine présentaient de façon inattendue d'excellentes propriétés anti-bactériennes alors que les mêmes esters d'autres hydrates de carbone tels que le glucose se sont avérés être dépourvus d'une telle activité.

La présente invention a pour objet, à titre de produits industriels nouveaux, des esters rétinoïques de D-désosamine, ceux-ci pouvant être représentés par la formule générale suivante:

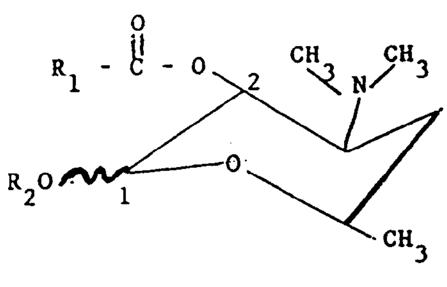

(I)

dans laquelle:

le radical $R_1$

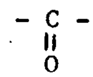

est soit le radical rétinoyle (all trans) ou (13 cis) soit le radical étrétinoyle, et

$R_2$ représente un radical alkyle, linéaire ou ramifié, ayant de 1 à 24 atomes de carbone,

et les anomères α et β et leurs mélanges ainsi que les sels des composés de formule (I).

Parmi les radicaux alkyles, linéaires ou ramifiés ayant de 1 à 24 atomes de carbone, on peut notamment mentionner les radicaux méthyle, éthyle, propyle, isopropyle, butyle, tert-butyle, octyle, nonyle, décyle, undécyle, dodécyle, tétradécyle, hexadécyle, octadécyle et décyl-2 tétradécyle.

Lorsque les esters rétinoïques de D-désosamine dérivent de l'acide étrétinique, ceux-ci peuvent être représentés par la formule générale suivante:

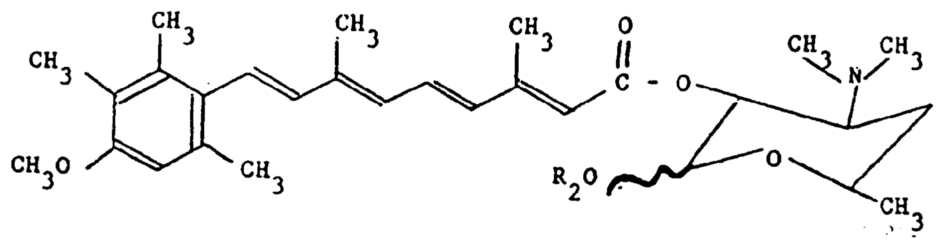

(II)

dans laquelle:

$R_2$ a la même signification que celle donnée ci-dessus pour la formule (I).

Lorsque les esters rétinoïques de D-désosamine dérivent de l'acide rétinoïque (all trans) ou (13 cis), ceux-ci peuvent être représentés par la formule générale suivante:

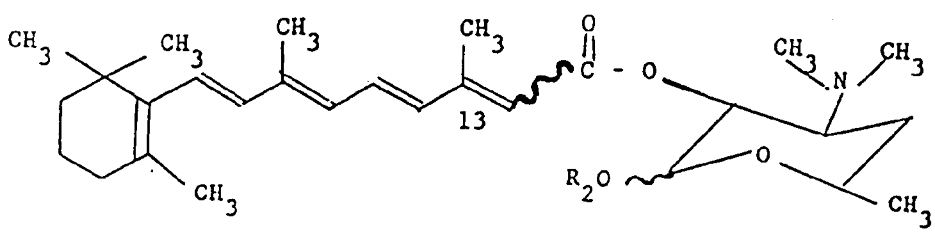

(III)

dans laquelle:

le radical $R_2$ a la même signification que celle donnée ci-dessus pour la formule générale (I).

Parmi les esters rétinoïques de D-désosamine selon l'invention on peut notamment mentionner les suivants:

1) O-Rétinoyl (13 cis)-2-O-butyl-1-α,β-D-désosamine,
2) O-Rétinoyl (all trans)-2-O-butyl-1-α,β-D-désosamine,
3) O-Rétinoyl (all trans)-2-O-butyl-1-α-D-désosamine,
4) O-Rétinoyl (13 cis)-2-O-butyl-1-α-D-désosamine,
5) O-Etrétinoyl (all trans)-2-O-butyl-1-α,β-D-désosamine,
6) O-Etrétinoyl (all trans)-2-O-butyl-1-α-D-désosamine,
7) O-Etrétinoyl (all trans)-2-O-butyl-1-β-D-désosamine,
8) O-Rétinoyl (13 cis)-2-O-méthyl-1- α,β-D-désosamine,
9) O-Rétinoyl (all trans)-2-O-décyl-2'-tétradécyl-1-α,β-D-désosamine,
10) O-Rétinoyl (13 cis)-2-O-décyl-2'-tétradécyl-1-α-D-désosamine.

La présente invention a également pour objet le procédé de préparation des esters rétinoïques de D-désosamine tels que définis ci-dessus.

Différents procédés d'estérification de la D-désosamine, en position 2, peuvent être utilisés mais de préférence celle-ci est réalisée en milieu solvant organique anhydre comme le tétrahydrofuranne, seul ou en mélange avec un autre solvant organique tel que la pyridine ou le N,N-diméthylformamide, en faisant réagir un excès d'anhydride mixte, soit de l'acide étrétinique, soit de l'acide rétinoïque (all trans) ou (13 cis), préparé in situ, par exemple à partir de chloroformiate d'éthyle et de l'acide choisi, sur un monoéther en position (1) de la D-désosamine.

D'autres méthodes d'estérification peuvent être employées, notamment la méthode utilisant les imidazolides des acides choisis, dans un solvant anhydre comme la pyridine ou le N,N-diméthylformide, en présence d'une base comme le tert-butanolate de potassium ou l'imidazolidure de sodium, mais ces méthodes donnent de manière générale des rendements plus faibles.

Les monoéthers en position 1 de la D-désosamine sont obtenus par les méthodes conventionnelles de glycosylation en faisant réagir sur la D-désosamine, l'alcool choisi ($R_2$ OH) en présence d'un acide minéral tel que l'acide sulfurique ou chlorhydrique ou d'un acide organique tel que l'acide paratoluène sulfonique, éventuellement dans un solvant organique tel que le N,N-diméthylformamide à une température d'environ 80°C.

Les composés selon l'invention conviennent tout particulièrement aux domaines suivants:

1) pour traiter des affections dermatologiques liées à un désordre de la kératinisation portant sur la différenciation et sur la prolifération notamment pour traiter les acnés vulgaires, comédoniennes, polymorphes, les acnés nodulo kystiques, conglobata, les acnés séniles, les acnés secondaires telles que l'acné solaire, médicamenteuse, professionnelle;

2) pour traiter d'autres types de trouble de la kératinisation, notamment les ichtyoses, les états ichtyosiformes, la maladie de Darier, les kératodermies palmoplantaires, les leucoplasies et les états leucoplasiformes, le lichen;

3) pour traiter d'autres affections dermatologiques liées à un trouble de la kératinisation avec une composante inflammatoire et/ou immunoallergique et, notamment, toutes les formes de psoriasis qu'il soit cutané, muqueux ou unguéal, et même le rhumatisme psoriasique ou encore l'atopie cutanée, telle que l'eczéma ou l'atopie respiratoire, ces composés peuvent également être utilisés dans certaines affections inflammatoires ne présentant pas de trouble de la kératinisation;

4) pour traiter toutes les proliférations dermiques ou épidermiques qu'elles soient bénignes ou malignes, qu'elles soient d'origine virale telle que verrues vulgaires, les verrues planes et l'épidermodysplasie verruciforme, les proliférations pouvant également être induites par les ultra-violets notamment dans le cadre des épithélioma baso et spino cellulaires;

5) pour traiter d'autres désordres dermatologiques tels que les dermatoses bulleuses et les maladies du collagène;

6) pour traiter certains troubles ophtalmologiques, notamment les cornéopathies;

7) pour lutter contre le vieillissement de la peau, qu'il soit photo-induit ou non;

8) pour prévenir ou guérir les stigmates de l'atrophie épidermique et/ou dermique induite par les corticostéroïdes locaux ou systémiques, ou toute autre forme d'atrophie cutanée.

La présente invention a donc pour objet l'utilisation des esters rétinoïques de D-désosamine tels que définis ci-dessus en tant qu'agents actifs pour des compositions à usage thérapeutique.

Les compositions thérapeutiques destinées notamment au traitement des affections ci-dessus mentionnées, contiennent, dans un support pharmaceutique acceptable, au moins un ester rétinoïque de D-désosamine ou un de ses sels tel que défini ci-dessus par la formule générale (I).

Ces esters rétinoïques de D-désosamine sont généralement administrés à une dose journalière d'environ 0,01mg/kg à 50mg/kg de poids corporel.

Comme support des compositions on peut utiliser tout support conventionnel, le composé actif se trouvant soit à l'état dissous soit à l'état dispersé dans le véhicule.

L'administration peut être effectuée par voie entérale, parentérale, topique ou oculaire. Par voie entérale les compositions thérapeutiques peuvent se présenter sous forme de comprimés, de gelules, de dragées, de sirops, de suspensions, de solutions, de poudres, de granulés, d'émulsions. Par voie parentérale, les compositions peuvent se présenter sous forme de solutions ou suspensions pour perfusions ou pour injections.

Par voie topique, les compositions thérapeutiques à base des esters rétinoïques de D-désosamine selon l'invention se présentent sous forme d'onguents, de teintures, de crèmes, de pommades, de poudres, de timbres, de tampons imbibés, de solutions, de lotions, de gels, de sprays ou encore de suspensions.

Par voie oculaire, ce sont principalement des collyres.

Ces compositions thérapeutiques contiennent au moins un ester rétinoïque de D-désosamine tel que défini ci-dessus à une concentration de préférence comprise entre 0,001 et 5% par rapport au poids total de la composition.

Les esters rétinoïques de D-désosamine de formule générale (I) trouvent également une application dans le domaine cosmétique en particulier dans l'hygiène corporelle et capillaire et notamment pour le traitement des peaux à tendance acnéïque, pour la repousse des cheveux, l'anti-chute, pour lutter contre l'aspect gras

de la peau ou des cheveux, dans la protection contre les effets néfastes du soleil, dans le traitement des peaux physiologiquement sèches ou comme déodorants.

La présente invention vise donc également des compositions cosmétiques contenant dans un support cosmétique acceptable, au moins un ester rétinoïque de D-désosamine ou un de ses sels de formule générale (I), cette composition se présentant notamment sous forme d'une lotion, d'un gel, d'un savon, d'un shampooing, d'un stick, d'un spray ou d'une mousse aérosols.

La concentration en ester rétinoïque de D-désosamine de formule générale (I) dans les compositions cosmétiques est généralement comprise entre 0,0001 et 5% en poids et de préférence entre 0,001 et 3% en poids.

Les compositions thérapeutiques et cosmétiques selon l'invention peuvent en outre contenir des additifs inertes ou même pharmacodynamiquement ou cosmétiquement actifs et notamment: des agents hydratants comme la thiamorpholinone et ses dérivés ou l'urée; des agents anti-séborrhéïques ou anti-acnéiques, tels que la S-carboxyméthylcystéine, la S-benzyl-cystéamine et leurs sels et leurs dérivés, la thioxolone ou le peroxyde de benzoyle, des antibiotiques comme l'érythromycine et ses esters, la néomycine, les tétracyclines ou les polyméthylène-4,5 isothiazolinones-3; des agents favorisant la repousse des cheveux, comme le "Minoxidil" (2,4-diamino-6-pipéridino-pyrimidine-3-oxyde) et ses dérivés, le Diazoxide (7-chloro-3-méthyl-1,2,4-benzothiadiazine-1,1-dioxyde) et le Phénytoïn (5,5-diphénylimidazolidine-2,4-dione); des agents anti-inflammatoires stéroïdiens et non-stéroïdiens; des caroténoïdes et notamment le β-carotène; des agents anti-psoriasiques tels que l'anthraline et ses dérivés et les acides eicosatétraynoïque-5,8,11,14 et triynoïque-5,8,11 leurs esters et leurs amides.

Les compositions selon l'invention qu'elles soient à usage thérapeutique ou cosmétique peuvent également contenir des agents d'amélioration de la saveur, des agents conservateurs, des agents stabilisants, des agents régulateurs d'humidité, des agents régulateurs de pH, des agents modificateurs de pression osmotique, des agents émulsionnants, des filtres UV-A et UV-B, des antioxydants tels que l'α-tocophérol, le butylhydroxyanisole ou le butylhydroxytoluène.

Lorsque les compositions selon l'invention sont destinées à un traitement anti-bactérien, celui-ci consiste à en appliquer une quantité suffisante 2 à 3 fois par jour, sur les zones de la peau à traiter et ceci pendant une période de temps de 6 à 30 semaines et de préférence de 12 à 24 semaines.

Les compositions anti-bactériennes selon l'invention peuvent également être utilisées à titre préventif c'est-à-dire sur les zones de peau susceptibles d'être atteintes d'une prolifération bactérienne.

Bien qu'il ait été fait référence plus particulièrement au domaine thérapeutique et cosmétique, les esters rétinoïques de D-désosamine selon l'invention peuvent également être utilisés comme agents anti-bactériens et antiseptiques dans d'autres domaines de l'industrie telle que l'agriculture, la papeterie, les peintures et vernis, le traitement des eaux, etc...

## ETUDES COMPARATIVES SUR L'ACTIVITE DES ESTERS RETINOIQUES DE D-DESOSAMINE.

L'activité des esters rétinoïques de D-désosamine a été étudiée par la méthode de dilution en vue de déterminer la concentration minimale inhibitrice (CMI), méthode décrite et employée par G. A. DENYS et al, Antimicrobial Agents and Chemotherapy (1983) 23 335-337 et J.J. LEYDEN et al, J. Am. Acad. Dermatol. (1983) 8 (1) 41-5, en utilisant comme souche de propionibactérium acnes, la souche P37 fournie par CUNLIFFE et HOLLAND. Cette souche P37 a fait l'objet des études décrites dans les publications suivantes:

- J. GREENMAN, K.T. HOLLAND et W.J. CUNLIFFE, Journal of General Microbiology (1983) 129, 1301-1307,
- E. INGHAM, K.T. HOLLAND, G. GOWLAND et W.J. CUNLIFFE, ibid (1980) 118, 59-65 et
- K.T. HOLLAND, J. GREENMAN et W.J. CUNLIFFE, Journal of Applied Bacteriology (1979) 47, 383-394.

### Sélection et isolement des populations sensibles et résistantes.

La souche P37 est sensible à l'érythromycine comme le montre la concentration minimale inhibitrice (CMI=0,78μg/ml).

En revanche, après 8 sous-cultures successives dans le même milieu, (RCM* 19/20, DMSO 1/20 en volume) en vue d'obtenir une stabilisation progressive de cette souche à ce milieu, une résistance progressive à l'érythromycine se manifeste sous la forme suivante:

- après étalage d'un inoculum standardisé (DO=1,8 à 450nm) sur milieu gélosé (RCM + furazolidone), en boîte de Petri, un disque de 9mm de diamètre est déposé au centre de celle-ci. Sur le disque, 50μg d'érythromycine (en solution dans le DMSO) sont déposés.

* Reinforced Clostridium Meium (OXOID).

- après 6 jours à 36°C en milieu anaérobie (système GAS-PAK, B.B.L) une zone d'inhibition de la pousse de la souche est nettement visible (diamètre total= 42mm), la majorité des colonies étant située à la périphérie de la zone d'inhibition.

En revanche à l'intérieur de celle-ci quelques colonies apparaissent nettement.

Les deux types de colonies sont alors prélevés par arrachage du milieu gélosé (Anse de platine stérilisé):

1) à l'intérieur de la zone d'inhibition on prélève les souches dénommées P37 $E^{\ominus}$ en raison de leur résistance apparente à l'érythromycine.

2) à 1cm au-delà de la périphérie de la zone d'inhibition, on prélève les souches dénommées P37 $E^{\oplus}$.

Après isolement et culture, les souches P37 $E^{\oplus}$ et P37 $E^{\ominus}$ montrent effectivement des sensibilités très différentes à l'érythromycine illustrées par les valeurs suivantes des CMI respectives.

| | CMI (µg/ml) |
|---|---|
| P37 | 0,78 |
| P37 $E^{\oplus}$ | 0,78 |
| P37 $E^{\ominus}$ | 50 |

Ce phénomène est confirmé par l'étude de la CI 50 (concentration inhibitrice à 50%) qui représente la concentration d'érythromycine où, à un temps constant de culture, 50% de survivants parmi la population sont retrouvés.

| | CI 50 (µg/ml) |
|---|---|
| P37 | 50 |
| P37 $E^{\oplus}$ | 5 |
| P37 $E^{\ominus}$ | 100 |

La concentration minimale inhibitrice (CMI) exprimée en µg/ml des esters rétinoïques de D-désosamine testés vis-à-vis des souches de propionibactérium Acnes P37, P37 $E^{\oplus}$ et P37 $E^{\ominus}$ et de staphilococcus epidermidis ATCC 12228 est reportée dans le tableau I suivant:

TABLEAU I

| Esters rétinoïques de D-désosamine composés N° (voir page 3) | P37 | P37 E⊕ | P37 E⊖ | ATCC 12228 |
|---|---|---|---|---|
| 1 | 10,5 | 21 | 10,5 | 18,5 |
| 2 | 15 | 25 | 9 | 3,5 |
| 5 | 3 | 6 | 6 | 6 |
| 6 | 19 | 4,3 | 2,1 | 1,5 |
| 7 | 5,2 | 40 | 14,5 | 2,3 |
| Composés de référence | | | | |
| α, β-D-désosamine, | >50 | >70 | >70 | >50 |
| O-butyl-1-α, β-D-désosamine, | >100 | >110 | >110 | >110 |
| O-rétinoyl (13-cis)-6-O-méthyl-1-α-D-glucopyrannose. | >45 | >45 | >45 | >45 |

Comme on peut le constater d'après les valeurs obtenues, les esters rétinoïques de D-désosamine selon l'invention présentent une excellente activité vis-à-vis des souches de Propionibacterium Acnes et de Staphilococcus epidermidis alors que les composés de référence sont totalement inactifs.

EXEMPLES DE PREPARATION

EXEMPLE 1

Préparation du O-rétinoyl (13 cis)-2-O-butyl-1-α,β-D-désosamine

Dans un ballon, sous atmosphère inerte, on dissout 5g (16,6mmoles) d'acide rétinoïque (13-cis) dans 35ml de tétrahydrofuranne anhydre; le mélange réactionnel est refroidi à 0°C puis on verse 3ml de pyridine anhydre et 1,6ml (16,6mmoles) de chloroformiate d'éthyle. La solution est agitée 5 minutes et on ajoute 2,5g (30mmoles) d'hydrogénocarbonate de sodium puis 1,5g (6,5mmoles) de O-butyl-α,β-D-désosamine préalablement dissous dans 150ml de tétrahydrofuranne. Le mélange réactionnel est alors laissé sous agitation pendant 10 heures en laissant remonter à température ambiante (chromatographie sur couche mince de gel de silice: chlorure de méthylène/méthanol 10%). La solution est versée sur 60ml d'eau puis extraite à l'acétate d'éthyle. La phase organique est séchée sur sulfate de magnésium, filtrée puis concentrée sous vide partiel. Le produit brut ainsi obtenu est chromatographié sur colonne de gel de silice (H.P.L.C.) en utilisant l'éluant: acétate d'éthyle (5)/hexane (5) pour aboutir à l'isolement de 2,5g (75% de rendement) du mélange d'anomères , du O-rétinoyl (13 cis) O-butyl-D-désosamine.

Microanalyse: $C_{32}H_{51}NO_4$, $2H_2O$; M:549,9

| | C | H | N |
|---|---|---|---|
| Calc% : | 69,88 | 10,09 | 2,54 |
| Tr% : | 70,25 | 9,65 | 2,45 |

Infra-rouge : bande à 1735cm$^{-1}$ (ester)

R.M.N. du $^{13}C$ ($CDCl_3$, réf. interne T.M.S.)

Effets $\gamma$ négatifs en 1 (-3ppm) et 3 (-2,8ppm) indiquent la position de l'ester en 2 pour l'anomère $\alpha$ (C-1:96,5ppm, C-3:57,50ppm); effets $\gamma$ négatifs en 1 (-2,5ppm) et en 3 (-2,3ppm) indiquent également la position de l'ester en 2 pour l'anomère $\beta$ (C-1:102,55ppm), C-3:63,07ppm). Le carbone $C'_{14}$ à 116,8ppm indique que la chaîne rétinoïque est de configuration 13 cis.

EXEMPLE 2

Préparation du O-rétinoyl (all trans)-2-O-butyl-1-$\alpha,\beta$ D-désosamine

Dans un ballon, sous atmosphère inerte on dissout 5g (16,6mmoles) d'acide rétinoïque (all trans) dans 35ml de tétrahydrofuranne anhydre; le mélange réactionnel est refroidi à 0°C puis on verse 3ml (38mmoles) de pyridine anhydre et 1,6ml (16,6mmoles) de chloroformiate d'éthyle; la solution est agitée 5 minutes et on ajoute 2,5g (30mmoles) d'hydrogénocarbonate de sodium puis 1,5g (6,5mmoles) de O-butyl-$\alpha,\beta$-D-désosamine préalablement dissous dans 150ml de tétrahydrofuranne. Le mélange réactionnel est alors laissé sous agitation pendant 10 heures en laissant remonter à température ambiante (chromatographie sur couche mince de gel de silice: chlorure de méthylène/méthanol 10%). La solution est versée sur 60ml d'eau puis extraite à l'acétate d'éthyle. La phase organique est séchée sur sulfate de magnésium, filtrée puis concentrée sous vide partiel. Le produit brut ainsi obtenu est chromatographié sur colonne de gel de silice (H.P.L.C.) en utilisant l'éluant: acétate d'éthyle (5)/hexane (5) pour aboutir à l'isolement de 2,7g (81% de rendement) du mélange d'anomères $\alpha,\beta$ du O-rétinoyl (all trans)-2-O-butyl-1-D-désosamine.

Microanalyse : $C_{32}H_{51}NO_4$, $2H_2O$; M:549,9

| | C | H | N |
|---|---|---|---|
| Calc % : | 69,88 | 10,09 | 2,54 |
| Tr % : | 69,82 | 9,82 | 2,44 |

R.M.N. du $^{13}C$ ($CDCl_3$, réf. interne T.M.S.).

Confirmation de la stéréochimie de la chaîne all trans avec le carbone $C'_{14}$ à 118,7ppm sans trace de chaîne 13 cis.

EXEMPLE 3

Préparation du O-rétinoyl (all trans)-2-O-butyl-1$\alpha$ -D-désosamine

Dans un ballon, sous atmosphère inerte on dissout 5g (16,6mmoles) d'acide rétinoïque (all trans) dans 35ml de tétrahydrofuranne anhydre. Le mélange réactionnel est refroidi à 0°C puis on verse 3ml (38mmoles) de pyridine anhydre et 1,6ml (16,6mmoles) de chloroformiate d'éthyle; la solution est agitée 5 minutes et on ajoute 2,5g (30mmoles) d'hydrogénocarbonate de sodium puis 1,5g (6,5mmoles) de O-butyl-$\alpha$-D-désosamine préalablement dissous dans 150ml de tétrahydrofuranne. Le mélange réactionnel est alors laissé sous agitation pendant 10 heures en laissant remonter à température ambiante (chromatographie sur couche mince de gel de silice : chlorure de méthylène/méthanol 10%). La solution est versée sur 60ml d'eau puis extraite à l'acétate d'éthyle. La phase organique est séchée sur sulfate de magnésium, filtrée puis concentrée sous vide partiel. Le produit brut ainsi obtenu est chromatographié sur colonne de gel de silice (H.P.L.C.) en utilisant l'éluant : acétate d'éthyle (5)/hexane (5) pour aboutir à l'isolement de 2,3g (69% de rendement) de O-rétinoyl (all trans)-2-O-butyl-1-$\alpha$-D-désosamine pur.

Microanalyse : $C_{32}H_{51}NO_4$, 1,5$H_2O$; M: 540,9

| | C | H | N |
|---|---|---|---|
| Calc % : | 71,04 | 10,05 | 2,58 |
| Tr % : | 70,90 | 9,59 | 2,51 |

R.M.N. du $^{13}C$ ($CDCl_3$, réf. interne T.M.S.)

Effets γ négatifs en 1 (-2,9ppm) et 3 (-2,6ppm) indiquent la position de l'ester en 2. Le déplacement chimique du $C'_{14}$ (118,65ppm) est en accord avec la stéréochimie all trans de la chaîne rétinoïque.

EXEMPLE 4

Préparation du O-rétinoyl (13 cis)-2-O-butyl-1 α-D-désosamine

Dans un ballon, sous atmosphère inerte, on dissout 3g (10mmoles) d'acide rétinoïque (13 cis) dans 25ml de tétrahydrofuranne anhydre; le mélange réactionnel est refroidi à 0°C puis on verse 2ml de pyridine anhydre et 1 ml (10mmoles) de chloroformiate d'éthyle. La solution est agitée 5 minutes et on ajoute 2g d'hydrogénocarbonate de sodium puis 1g (4,3mmoles) de O-butyl-α-D-désosamine préalablement dissous dans 100ml de tétrahydrofuranne. Le mélange réactionnel est alors laissé sous agitation pendant 10 heures en laissant remonter à température ambiante (chromatographie sur couche mince de gel de silice: chlorure de méthylène/méthanol 10%). La solution est versée sur 60ml d'eau puis extraite à l'acétate d'éthyle. La phase organique est séchée sur sulfate de magnésium, filtrée puis concentrée sous vide partiel. Le produit brut ainsi obtenu est chromatographié sur colonne de gel de silice (H.P.L.C.) en utilisant l'éluant: acétate d'éthyle (5)/hexane (5) pour aboutir à l'isolement de 1,5g (67% de rendement) de O-rétinoyl (13 cis)-2-O-butyl-1-α-D-désosamine pur.

Microanalyse: $C_{32}H_{51}NO_4$, 1,5$H_2O$; M: 540,9

| | C | H | N |
|---|---|---|---|
| Calc % : | 71,04 | 10,05 | 2,58 |
| Tr % : | 71,05 | 9,47 | 2,52 |

Infra-rouge : bande à 1735$cm^{-1}$ (ester)

R.M.N. du $^{13}C$ ($CDCl_3$, réf. interne T.M.S.)

Effets négatifs en 1 (-3ppm) et 3 (-2,8ppm) indiquent la position de l'ester en 2. Le carbone $C'_{14}$ (116,48ppm) de la chaîne rétinoïque est en accord avec la stéréochimie 13 cis de la chaîne rétinoïque.

EXEMPLE 5

Préparation du O-étrétinoyl (all trans)-2-O-butyl-1-α,β-D-désosamine

Dans un ballon, sous atmosphère inerte, on dissout 5g (15,3mmoles) d'acide étrétinique (all trans) dans 35ml de tétrahydrofuranne anhydre; le mélange réactionnel est refroidi à 0°C puis on verse 3ml de pyridine anhydre et 1,5ml (15,3mmoles) de chloroformiate d'éthyle. La solution est agitée 5 minutes et on ajoute 2,5g d'hydrogénocarbonate de sodium puis 1,5g (6,5mmoles) de O-butyl-α,β-D-désosamine préalablement dissous dans 150ml de tétrahydrofuranne. Le mélange réactionnel est alors laissé sous agitation pendant 10 heures en laissant remonter à température ambiante (chromatographie sur couche mince de gel de silice: chlorure de méthylène/méthanol 10%). La solution est versée sur 60ml d'eau puis extraite à l'acétate d'éthyle. La phase organique est séchée sur sulfate de magnésium, filtrée puis concentrée sous vide partiel. Le produit brut ainsi obtenu est chromatographié sur colonne de gel de silice (H.P.L.C.) en utilisant l'éluant: acétate d'éthyle (5)/hexane (5) pour aboutir à l'isolement de 2,7g (78% de rendement) de O-étrétinoyl (all trans)-2-O-butyl-1α,β-D-désosamine.

Microanalyse: $C_{33}H_{49}NO_5$, $1,5H_2O$; M: 566,9

| | C | H | N |
|---|---|---|---|
| Calc % : | 69,9 | 9,24 | 2,47 |
| Tr % : | 69,55 | 8,95 | 2,41 |

R.M.N. du $^{13}C$ ($CDCl_3$, réf. interne T.M.S.)

Les effets γ négatifs en 1 (-3,3ppm) et 3 (-2,6ppm) indiquent la position de l'ester en 2 pour l'anomère α ainsi que les effets γ négatifs en 1 (-2,3ppm) et 3 (-2,3ppm) pour l'anomère β.

Le déplacement chimique du $C'_{14}$ (119,15ppm) est en accord avec la configuration all trans majoritaire de la chaîne alors qu'une trace de chaîne 13 cis est décelée à 117,09ppm ($C'_{14}$).

EXEMPLE 6

Préparation du O-étrétinoyl (all trans)-2-O-butyl-1-α -D-désosamine

Dans un ballon, sous atmosphère inerte, on dissout 5g (15,3mmoles) d'acide étrétinique (all trans) dans 35ml de tétrahydrofuranne anhydre; le mélange réactionnel est refroidi à 0°C puis on verse 3ml de pyridine anhydre et 1,5ml (15,3mmoles) de chloroformiate d'éthyle. La solution est agitée 5 minutes et on ajoute 2,5g d'hydrogénocarbonate de sodium puis 1,5g (6, 5mmoles) de O-butyl-α-D-désosamine préalablement dissous dans 150ml de tétrahydrofuranne. Le mélange réactionnel est alors laissé sous agitation pendant 10 heures en laissant remonter à température ambiante (chromatographie sur couche mince de gel de silice: chlorure de méthylène/méthanol 10%). La solution est versée sur 60ml d'eau puis extraite à l'acétate d'éthyle. La phase organique est séchée sur sulfate de magnésium, filtrée puis concentrée sous vide partiel. Le produit brut ainsi obtenu est chromatographié sur colonne de gel de silice (H.P.L.C.) en utilisant l'éluant: acétate d'éthyle (5)/hexane (5) pour aboutir à l'isolement de 2,5g (72% de rendement) de O-étrétinoyl (all trans)-2-O-butyl-1-α-D-désosamine pur.

Microanalyse : $C_{33}H_{49}NO_5$, $0,5H_2O$; M: 548,8

| | C | H | N |
|---|---|---|---|
| Calc % : | 72,22 | 9,18 | 2,55 |
| Tr % : | 72,47 | 9,25 | 2,16 |

R.M.N. du $^{13}C$ ($CDCl_3$, réf. interne T.M.S.).

Effets γ négatifs en 1 (-3,1ppm) et 3 (-2,8ppm) indiquent la position de l'ester en 2. La configuration all trans est confirmée par le déplacement chimique du $C'_{14}$ à 118,75ppm.

EXEMPLE 7

Préparation du O-étrétinoyl (all trans)-2-O-butyl-1-β-D-désosamine

Dans un ballon, sous atmosphère inerte, on dissout 5g (15,3mmoles) d'acide étrétinique all trans dans 35ml de tétrahydrofuranne anhydre; le mélange réactionnel est refroidi à 0°C puis on verse 3ml de pyridine anhydre et 1,5ml (15,3mmoles) de chloroformiate d'éthyle. La solution est agitée 5 minutes et on ajoute 2,5g d'hydrogénocarbonate de sodium puis 1,5g (6,5mmoles) de O-butyl-β-D-désosamine préalablement dissous dans 150ml de tétrahydrofuranne. Le mélange réactionnel est alors laissé sous agitation pendant 10 heures en laissant remonter à température ambiante (chromatographie sur couche mince de gel de silice : chlorure de méthylène/méthanol 10%). La solution est versée sur 60ml d'eau puis extraite à l'acétate d'éthyle. La phase organique est séchée sur sulfate de magnésium, filtrée puis concentrée sous vide partiel. Le produit brut ainsi obtenu est chromatographié sur colonne de gel de silice (H.P.L.C.) en utilisant l'éluant: acétate d'éthyle (5)/hexane (5) pour aboutir à l'isolement de 2,1g (60% de rendement) de O-étrétinoyl (all trans)-2-O-butyl-1-β-D-désosamine.

Microanalyse: $C_{33}H_{49}NO_5$, $1,5H_2O$; M: 566,9

| | C | H | N |
|---|---|---|---|
| Calc % : | 69,91 | 9,24 | 2,43 |
| Tr % : | 69,96 | 8,88 | 2,47 |

R.M.N. du $^{13}C$ ($CDCl_3$, réf. interne T.M.S.)

Effets γ négatifs en 1 (-2,4ppm) et 3 (-2,4ppm) indiquent la position de l'ester en 2. La configuration all trans de la chaîne est donnée par le déplacement chimique du $C'_{14}$ à 119,06ppm.

EXEMPLE 8

Préparation du O-Rétinoyl (13 cis)-2-O-décyl-2'-tétradécyl-1- -D-désosamine

Dans un ballon, sous atmosphère inerte, on introduit 217 mg (2mmoles) de chloroformiate d'éthyle et 20ml de tétrahydrofuranne. La solution est agitée et refroidie à - 5°C.

On ajoute alors goutte à goutte, sans dépasser 0°C une solution contenant 585 mg (1,94mmoles) d'acide rétinoïque 13-cis, 202mg (2mmoles) de triéthylamine et 20 ml de tétrahydrofuranne. Le milieu est agité encore 1heure 30 à température ambiante puis débarrassé des sels de triéthylamine par filtration.

Les filtrats sont alors introduits à température ambiante et sous atmosphère inerte dans un ballon contenant 250mg (0,48mmoles) de O-décyl-2'-tétradécyl-1-α-D-désosamine, 0,15ml de pyridine anhydre (1,3mmoles).

Le milieu réactionnel est laissé sous agitation pendant 15 heures.

La réaction est suivie par chromatographie sur couche mince de gel de silice en utilisant l'éluant : chlorure de méthylène (85)/méthanol (15).

Le milieu est alors concentré puis repris dans du toluène et purifié par chromatographie sur colonne de gel de silice (HPLC) en utilisant l'éluant : acétate d'éthyle (6)/heptane (4) pour aboutir à l'isolement de 75mg (18% de rendement) de O-rétinoyl (13cis)-2-O-décyl-2'-tétradécyl-1-α- D-désosamine accompagné d'une trace de son isomère all trans.

Microanalyse:

| | C | H | N |
|---|---|---|---|
| Calc % : | 78,63 | 11,55 | 1,76 |
| Tr % : | 78,12 | 11,45 | 1,84 |

RMN du $^{13}C$ ($CDCl_{13}$, ref. interne T.M.S.) : le spectre est en accord avec la structure proposée.

COMPOSITIONS PHARMACEUTIQUES ET COSMETIQUES

A - Gels pour le traitement topique de l'acné

| | |
|---|---|
| 1. Hydroxypropyl cellulose.................. | 1g |
| Butylhydroxytoluène...................... | 0,05g |
| O-rétinoyl (13 cis)-2-O-butyl-1-α,β-D-désosamine............................... | 0,1g |
| Isopropanol q.s.p......................... | 100g |

| | |
|---|---|
| 2. Hydroxypropyl cellulose.................. | 1,5g |
| Butylhydroxytoluène...................... | 0,05g |
| O-rétinoyl (all trans)-2-O-butyl-1-α,β-D-désosamine............................... | 0,075g |
| Isopropanol q.s.p......................... | 100g |

B - Lotion pour le traitement topique de l'acné

| | |
|---|---|
| Butylhydroxytoluène...................... | 0,05g |
| O-rétinoyl (13 cis)-2-O-butyl-1-α,β-D-désosamine............................... | 0,7g |
| Triglycérides d'acides gras en $C_8$-$C_{12}$ q.s.p................................... | 100g |

C - Stick pour le traitement de l'acné

| | |
|---|---|
| Vaseline blanche......................... | 52,7g |
| Huile de vaseline........................ | 15g |
| Paraffine raffinée....................... | 32g |
| O-rétinoyl (all trans)-2-O-butyl-1-α,β-D-désosamine............................... | 0,3g |

**Revendications**

1. Esters rétinoïques de D-désosamine, caractérisés par le fait qu'ils répondent à la formule générale suivante:

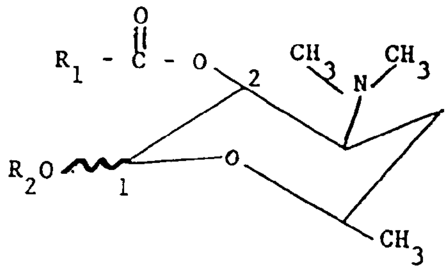

(I)

dans laquelle:

le radical $R_1$

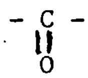

est soit le radical rétinoyle (all trans) ou (13 cis) soit le radical étrétinoyle, et

$R_2$ représente un radical alkyle, linéaire ou ramifié, ayant de 1 à24 atomes de carbone,

et les anomères α et β leurs mélanges ainsi que les sels des composés de formule (I).

2. Composés selon la revendication 1, caractérisés par le fait que le radical alkyle, linéaire ou ramifié, ayant de 1 à 24 atomes de carbone est le radical méthyle, éthyle, propyle, isopropyle, butyle, tert-butyle, octyle, nonyle, décyle, undécyle, dodécyle, tétradécyle, hexadécyle, octadécyle et décyl-2 tétradécyle.

3. Composés selon la revendication 1, caractérisés par le fait qu'ils dérivent de l'acide étrétinique et répondent à la formule générale suivante:

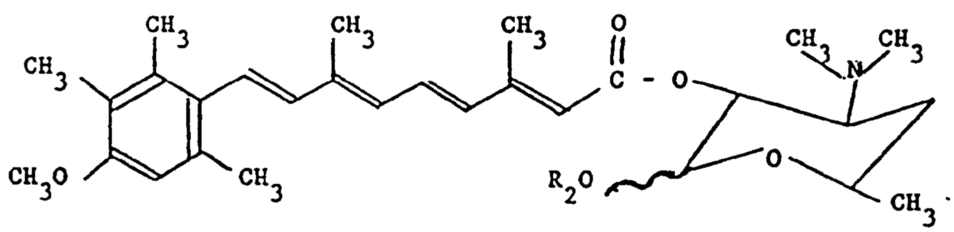

(II)

dans laquelle:

$R_2$ a la même signification que celle donnée à la revendication 1.

4. Composés selon la revendication 1, caractérisés par le fait qu'ils dérivent de l'acide rétinoïque (all trans) ou (13 cis) et répondent à la formule générale suivante:

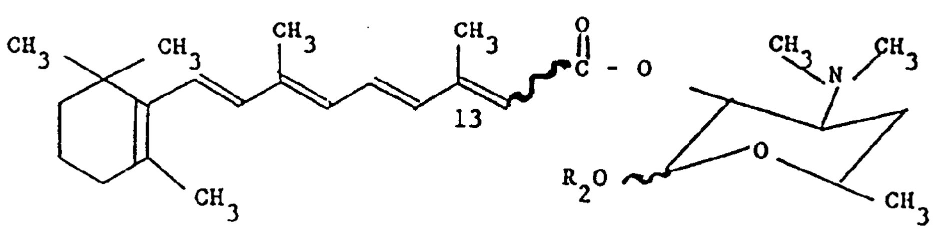

(III)

dans laquelle:

$R_2$ a la même signification que celle donnée à la revendication 1.

5. Composés selon l'une quelconque des revendications 1 à 4, caractérisés par le fait qu'ils sont pris dans le groupe constitué par:

O-Rétinoyl (13 cis)-2-O-butyl-1-α,β-D-désosamine,

O-Rétinoyl (all trans)-2-O-butyl-1-α,β-D-désosamine,
O-Rétinoyl (all trans)-2-O-butyl-1-α-D-désosamine,
O-Rétinoyl (13 cis)-2-O-butyl-1-α-D-désosamine,
O-Etrétinoyl (all trans)-2-O-butyl-1-α,β-D-désosamine,
O-Etrétinoyl (all trans)-2-O-butyl-1-α-D-désosamine,
O-Etrétinoyl (all trans)-2-O-butyl-1-β-D-désosamine,
O-Rétinoyl (13 cis)-2-O-méthyl-1-α,β-D-désosamine,
O-Rétinoyl (all trans)-2-O-décyl-2'-tétradécyl-1-α, β-D-désosamine,
O-Rétinoyl (13 cis)-2-O-décyl-2'-tétradécyl-1-α-D-désosamine.

6. Procédé de préparation des composés selon l'une quelconque des revendications 1 à 5, caractérisé par le fait qu'il consiste à faire réagir un excès d'anhydride mixte soit de l'acide étrétinique soit de l'acide rétinoïque (all trans) ou (13 cis), préparé in situ, sur un monoéther de D-désosamine en position (1), la réaction d'estérification étant effectuée en milieu solvant organique.

7. Procédé selon la revendication 6, caractérisé par le fait que le solvant organique est le tétrahydrofuranne, seul ou en mélange avec de la pyridine ou du N,N-diméthylformamide.

8. Composition pharmaceutique, caractérisée par le fait qu'elle contient dans un véhicule approprié pour une administration par voie entérale, parentérale, topique ou oculaire, au moins un composé de formule (I) selon l'une quelconque des revendications 1 à 5.

9. Composition selon la revendication 8, caractérisée par le fait qu'elle se présente sous une forme appropriée pour une application topique et contient de 0,001 à environ 5% en poids d'un composé de formule (I).

10. Utilisation d'un composé selon l'une quelconque des revendications 1 à 5, pour la préparation d'une composition pharmaceutique destinée au traitement des affections dermatologiques, rhumatismales, respiratoires ainsi qu'ophtalmologiques.

11. Composition cosmétique, caractérisée par le fait qu'elle contient, dans un véhicule cosmétique approprié, au moins un composé de formule (I) selon l'une quelconque des revendications 1 à 5.

12. Composition cosmétique selon la revendication 11, caractérisée par le fait qu'elle contient le composé de formule (I) à une concentration comprise entre 0,0001 et 5% et de préférence entre 0,001 et 3% en poids.

13. Utilisation des composés selon l'une quelconque des revendications 1 à 5 pour la préparation d'une composition destinée à lutter contre le vieillissement de la peau.

14. Utilisation des composés selon l'une quelconque des revendications 1 à 5 comme agents anti-bactériens dans l'industrie.

**Patentansprüche**

1. Retinsäureester von D-Desosamin, gekennzeichnet durch folgende allgemeine Formel:

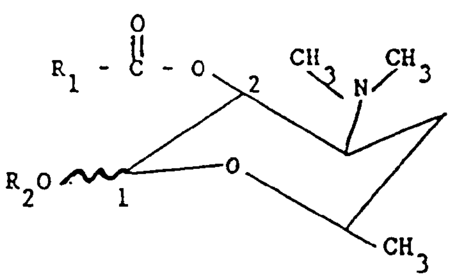

(I)

worin

das Radikal $R_1$-CO- entweder ein Retinsäurerest (all-trans oder 13-cis) oder ein Etretinsäurerest ist, und $R_2$ einen linearen oder verzweigten Alkylrest mit 1 bis 24 Kohlenstoffen darstellt sowie deren α- und β-Anomere und deren Gemische wie auch die Salze der Verbindungen der Formel (I).

2. Verbindungen gemäß Anspruch 1, dadurch gekennzeichnet, daß der lineare oder verzweigte Alkylrest mit 1 bis 24 Kohlenstoffatomen eine Methyl-, Ethyl-, Propyl-, Isopropyl-, Butyl-, tert.-Butyl-, Octyl-, Nonyl-, Decyl-, Undecyl-, Dodecyl-, Tetradecyl-, Hexadecyl-, Octadecyl- oder Decyl-2-tetradecylgruppe ist.

3. Verbindungen gemäß Anspruch 1, dadurch gekennzeichnet, daß sie Etretinsäurederivate der folgenden allgemeinen Formel sind:

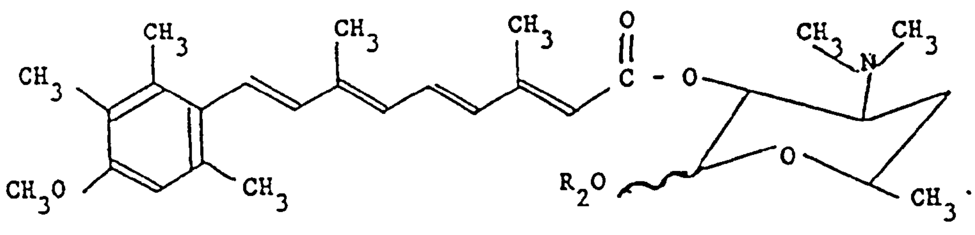

(II)

worin
$R_2$ die in Anspruch 1 angegebene Bedeutung hat.

4. Verbindungen gemäß Anspruch 1, dadurch gekennzeichnet, daß sie all-trans- oder 13-cis-Retinsäurederivate der folgenden allgemeinen Formel sind:

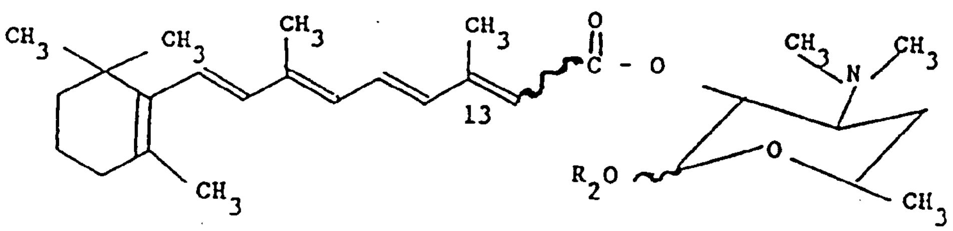

(III)

worin:
$R_2$ die in Anspruch 1 angegebene Bedeutung hat.

5. Verbindungen gemäß einem der Ansprüche 1 bis 4, dadurch gekennzeichnet, daß sie aus der folgenden Gruppe ausgewählt sind:
2-O-Butyl-1-α,β-D-desosamin-O-retinat (13-cis),
2-O-Butyl-α,β-D-desosamin-O-retinat (all-trans),
2-O-Butyl-α-D-desosamin-O-retinat (all-trans),
2-O-Butyl-1-α-D-desosamin-O-retinat (13-cis),
2-O-Butyl-1-α,β-D-desosamin-O-etretinat (all-trans),
2-O-Butyl-1-α-D-desosamin-O-etretinat (all-trans),
2-O-Butyl-1-β-D-desosamin-O-etretinat (all-trans),
2-O-Methyl-1 -α,β-D-desosamin-O-retinat (13-cis),
2-O-Decyl-2-tetradecyl-α,β-D-desosamin-O-retinat (all-trans) und
2-O-Decyl-2-tetradecyl-α-D-desosamin-O-retinat (13-cis)

6. Verfahren zur Herstellung einer Verbindung gemäß einem der Ansprüche 1 bis 5, dadurch gekennzeichnet, daß man einen Überschuß eines in-situ hergestellten gemischten Anhydrids sowohl der Etretinsäure als auch der all-trans- oder 13-cis-Retinsäure mit einem Monoether in der 1-Stellung des D-Desosamins reagieren läßt, wobei die Veresterung in einem organischen Lösungsmittel ausgeführt wird.

7. Verfahren gemäß Anspruch 6, dadurch gekennzeichnet, daß das Lösungsmittel Tetrahydrofuran allein oder im Gemisch mit Pyridin oder N,N-Dimethylformamid vorliegt.

8. Pharmazeutische Zubereitung, dadurch gekennzeichnet, daß sie außer einem zur enteralen, parenteralen, topischen oder ophthalmologischen Applikation geeigneten Träger mindestens eine Verbindung der Formel (I) gemäß einem der Ansprüche 1 bis 5 enthält.

9. Zubereitung gemäß Anspruch 8, dadurch gekennzeichnet, daß sie in einer für die topische Applikation geeigneten Form vorliegt und von 0,001 bis ungefähr 5 Gew.-% einer Verbindung gemäß der Formel (I) enthält.

10. Verwendung einer Verbindgung gemäß einem der Ansprüche 1 bis 5 zur Herstellung einer pharmazeutischen Zubereitung zur Behandlung dermatologischer oder rheumatischer Erkrankungen oder Erkrankungen der Atemwege sowie der Augen.

11. Kosmetische Zubereitung, dadurch gekennzeichnet, daß sie außer einem geeigneten kosmetischen Träger mindestens eine Verbindung der Formel (I) gemäß einem der Ansprüche 1 bis 5 enthält.

12. Kosmetische Zubereitung gemäß Anspruch 11, dadurch gekennzeichnet, daß sie eine Verbindung gemäß Anspruch 1 in einer Konzentration von 0,0001 und 5, vorzugsweise von 0,001 bis 3 Gew.-%, enthält.

13. Verwendung einer Verbindung gemäß einem der Ansprüche 1 bis 5 zur Herstellung einer Zubereitung zur Bekämpfung der Hautalterung.

14. Verwendung der Verbindungen gemäß einem der Ansprüche 1 bis 5 als antibakte rielle Mittel in der Industrie.

## Claims

1. Retinoic esters of D-desosamine, characterized in that they correspond to the following general formula:

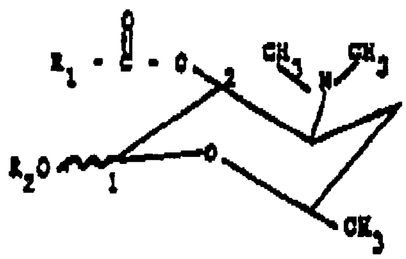

(I)

in which:

the radical $R_1$

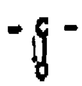

is either the all-trans- or 13-cis-retinoyl radical or the stratinoyl radical, and

$R_2$ represents a linear or branched alkyl radical having from 1 to 24 carbon atoms,

and the α and β anomers and their mixtures, as well as the salts of the compounds of formula (I).

2. Compounds according to Claim 1, characterized in that the linear or branched alkyl radical having 1 to 24 carbon atoms is the methyl, ethyl, propyl, isopropyl, butyl, tert-butyl, octyl, nonyl, decyl, undecyl, dodecyl, tetradecyl, hexadecyl, octadecyle or 2-decyltetradecyl radical.

3. Compounds according to Claim 1, characterized in that they are derived from etretinic acid and correspond to the following general formula:

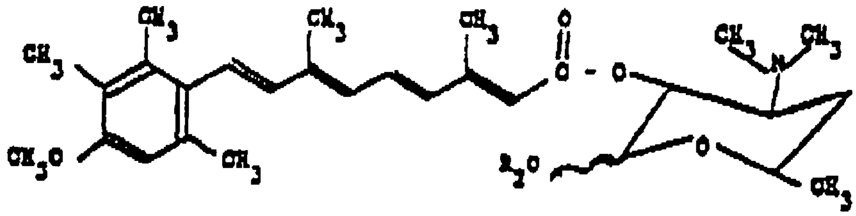

(II)

in which:

$R_2$ has the same meaning as that given in Claim 1.

4. Compounds according to Claim 1, characterized in that they are derived from all-trans- or 13-cis- retinoic acid and correspond to the following general formula:

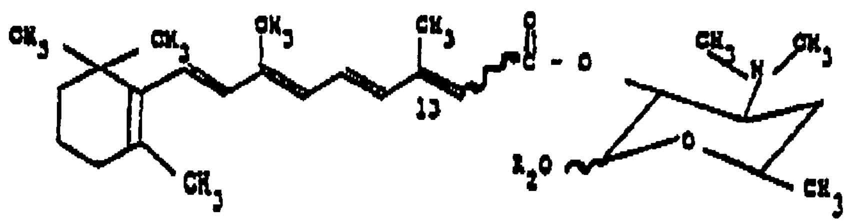

(III)

in which:

$R_2$ has the same meaning as that given in Claim 1.

5. Compounds according to any one of Claims 1 to 4, characterized in that they are taken from the group consisting of:

2-O-(13-cis-retinoyl)-1-O-butyl-α,βD-desosamine,
2-O-(all-trans-retinoyl)-1-O-bubyl-α,β-D-desosamine,
2-O-(all-trans-retinoyl)-1-O-butyl-α-D-desosamine,
2-O-(13-cis-retinoyl)-1-O-butyl-α-D-desosamine,
2-O-(all-trans-etretinoyl)-1-O-butyl-α-,β-D-desosamine,
2-O-(all-trans-etretinoyl)-1-O-butyl-α-D-desosamine,
2-O-(all-trans-etretinoyl)-1-O-butyl-β-D-desosamine,
2-O-(13-cis-retinoyl)-1-O-methyl-α,β-D-desosamine,
2-O-(all-trans-retinoyl)-2'-O-decyl-1-tetradecyl-α,β-D-desosamine,
2-O-(13-cis- retinoyl)-2'-O-decyl-1-tetradecyl-α-D-desosamine.

6. Process for the preparation of compounds according to any one of Claims 1 to 5, characterized in that it consists in reacting an excess of mixed anhydride either of etretinic acid or of all-trans- or 13-cis-retinoic acid, prepared in situ, with a D-desosamine monoether in position (1), the esterification reaction being performed in an organic solvent medium.

7. Process according to Claim 6, characterized in that the organic solvent is tetrahydrofuran, alone or mixed with pyridine or N,N-dimethylformamide.

8. Pharmaceutical composition, characterized in that it contains, in a vehicle suitable for administration by the enteral, parenteral, topical or ocular route, at least one compound of formula (I) according to any one of Claims 1 to 5.

9. Composition according to Claim 8, characterized in that it is provided in a form suitable for topical application and contains from 0.001 to approximately 5 % by weight of a compound or formula (I).

10. Use of a compound according to any one of Claims 1 to 5, for the preparation of a pharmaceutical composition intended for the treatment of dermatological, rheumatic, respiratory and also ophthalmological complaints.

**11.** Cosmetic composition, characterized in that it contains, in a suitable cosmetic vehicle, at least one compound of formula (I) according to any one of Claims 1 to 5.

**12.** Cosmetic composition according to Claim 11, characterised in that it contains the compound of formula (I) at a concentration between 0.0001 and 5 %, and preferably between 0.001 and 3 % by weight.

**13.** Use of the compound according to any one of Claims 1 to 5 for the preparation of a composition intended to combat ageing of the skin.

**14.** Use of compounds according to any one of Claims 1 to 5 as antibacterial agents in industry.